# EUROPÄISCHE PATENTSCHRIFT

(11) **EP 3 322 692 B1**
(45) Veröffentlichungstag und Bekanntmachung des Hinweises auf die Patenterteilung: **03.07.2019**
(21) Anmeldenummer: 16736892.7
(22) Anmeldetag: 12.07.2016
(51) Int. Cl.: C07C 263/10

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 16.07.2015 EP 15176964
(43) Veröffentlichungstag der Anmeldung: 23.05.2018
(73) Patentinhaber: Covestro Deutschland AG, 51373 Leverkusen (DE)
(72) Erfinder: STEFFENS, Friedhelm, 51373 Leverkusen (DE); MAHR, Bastian, 51467 Bergisch Gladbach (DE)
(74) Vertreter: Levpat
(86) Internationale Anmeldenummer: PCT/EP2016/066474
(87) Internationale Veröffentlichungsnummer: WO 2017/009311

(56) Entgegenhaltungen:
- EP-A1- 2 093 215
- DE-A1- 10 027 779
- DE-A1- 10 260 084

## Beschreibung

Die Erfindung betrifft ein Verfahren zur Herstellung von Isocyanaten durch Umsetzung von primären Aminen mit Phosgen im stöchiometrischen Überschuss in der Gasphase, bei dem das überschüssige Phosgen anschließend zurückgewonnen und in die Umsetzung zurückgeführt wird. Die vorliegende Erfindung betrifft insbesondere ein Verfahren zur geregelten Rückführung des zurückgewonnenen Phosgens, und zwar insbesondere dann, wenn der zurückzuführende Phosgenstrom auf mehrere parallel betriebene Gasphasenreaktoren aufgeteilt wird.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit dem Phosgen kann sowohl in der Gasphase als auch in der Flüssigphase erfolgen. Bei diesen Synthesen fällt das überschüssige Phosgen in der Regel zumindest teilweise zusammen mit dem in der Reaktion freigesetzten gasförmigen Nebenprodukt Chlorwasserstoff an, so dass es für einen wirtschaftlichen Betrieb einer Isocyanatsynthese unabdingbar ist, das überschüssige Phosgen vom Nebenprodukt Chlorwasserstoff abzutrennen und in die Reaktion zurückzuführen. Insbesondere wenn der zurückzuführende Phosgenstrom dabei auf mehrere Reaktoren aufgeteilt wird, stellt die Sicherstellung des vorgesehenen Phosgenstroms zu jedem einzelnen Reaktor insbesondere unter instationären Betriebszuständen eine besondere technische Herausforderung dar, so dass der Sicherstellung eines ausreichenden und stabilen Drucks in der Phosgenerzeugung eine wesentliche Bedeutung zukommt.

Es sind aus dem Stand der Technik verschiedene Verfahren zur Herstellung von Isocyanaten durch Umsetzung von Aminen mit Phosgen in der Gasphase bekannt.

EP 2 093 215 A1 beschreibt ein Verfahren zur Herstellung von Isocyanaten durch Umsetzung der entsprechenden Amine mit Phosgen, Kondensation des dabei anfallenden Gasgemisches, Strippen der dabei erhaltenen flüssigen Phase und Rückführung des so flüssig zurückerhaltenen Lösungsmittels. Die gasförmigen Bestandteile werden anschließend in einer Absorption weiter aufgereinigt.

GB 737 442 beschreibt ein Verfahren zur Rückgewinnung von flüssigem Phosgen aus Chlorwasserstoff und Phosgen enthaltenden Gasmischungen, bei dem das Gasgemisch aufwärts durch einen auf -40 bis -60°C gekühlten Kühler strömt, wobei das Phosgen kondensiert und in einen Vorratstank abläuft. Diese Schrift erwähnt, dass das zurückgewonnene flüssige Phosgen auf Grund seines geringen Gehalts an Chlorwasserstoff von weniger als 0,7 Gew.-% ohne weitere Aufreinigung in Reaktionen mit Aminen eingesetzt werden kann. Sie macht jedoch keine Angaben dazu, wie das zurückgewonnene flüssige Phosgen auf wirtschaftlich vorteilhafte Weise in einer Gasphasenreaktion verwendet werden kann. Darüber hinaus ist nachteilig an dem offenbarten Verfahren, dass das den Kühler verlassende Chlorwasserstoff-Gas noch nennenswerte Mengen an Phosgen enthält, die somit für die Phosgenierreaktion verloren sind. Weiterhin nachteilig ist, dass die Kondensation auf sehr niedrigem und damit energetisch aufwendigem Temperaturniveau durchgeführt wird.

US 2 764 607 beschreibt ein Verfahren zur Rückgewinnung von Phosgen aus einer Gasmischung mit Chlorwasserstoff aus der Produktion von Chloroformaten. Dazu wird das den Kondensator, der auf den Reaktionskessel montiert ist, verlassende Phosgen-Chlorwasserstoff-Gasgemisch zunächst in Kontakt mit kaltem Lösemittel gebracht, wobei bevorzugt das Phosgen in dem Lösemittel absorbiert wird. Anschließend wird das absorbierte Phosgen zusammen mit dem partiell mit absorbierten Chlorwasserstoff kontinuierlich einer Destillationskolonne wieder von dem Lösemittel getrennt. Dazu wird der Zulauf zwischen Abtriebs- und Verstärkerteil zugeführt und mit Hilfe eines Kopfkondensators ein Rücklauf auf die Destillationskolonne erzeugt, der das am Kopf entnommene Gasgemisch vollständig von Lösungsmittel befreit. Aus dem erhaltenen Gasstrom wird das Phosgen vollständig verflüssigt und einem Lagerbehälter zugeführt. Nachteilig an dem offenbarten Verfahren ist der hohe Bedarf an Kühlleistung auf niedrigem Temperaturniveau.

Ein alternatives Verfahren zur Auftrennung von gasförmigen Gemischen aus Chlorwasserstoff und Phosgen beschreibt DE 102 600 84**.** Die Schrift offenbart ein Verfahren, bei dem das Phosgen unter erhöhtem Druck kondensiert und die kondensierte Phase in einem anschließenden Verfahrensschritt zur Entfernung des Chlorwasserstoffes gestrippt wird. Die Strippung ist notwendig, da sich aufgrund des erhöhten Druckes nennenswerte Mengen Chlorwasserstoff in dem Kondensat lösen und diese sich nach Lehre der Schrift in Phosgenierreaktionen nachteilig auswirken. Nachteilig an dem offenbarten Verfahren ist, dass aufgrund des herrschenden Kondensationsdruckes ein weiterer Verfahrensschritt zum Abtrennung des gelösten Chlorwasserstoffs notwendig ist. Das Dokument liefert keine Anleitung zur Rückgewinnung von gasförmigem Phosgen. Die Schrift beschreibt, dass die Chlorwasserstoff- Phosgen-Trennung unter hohem Druck durchgeführt werden kann, dieses jedoch das Sicherheitsrisiko erhöht. Zudem ist die Erzeugung von hohem Druck energetisch aufwendig. Als Alternative wird die Trennung bei sehr niedrigen Temperaturen beschrieben, die jedoch ebenfalls energetisch aufwendig ist und zudem zu hohen Gehalten an Chlorwasserstoff in der flüssigen, Phosgen enthaltenden Phase führt.

In US 3 544 611 wird ein Verfahren zur Herstellung von organischen Isocyanaten beschrieben. Die flüssige Reaktionslösung wird in den mittleren Teil einer Destillationskolonne gespeist, um Chlorwasserstoff über Kopf und Phosgen und Isocyanat im Sumpf zu entnehmen. Der Sumpfstrom wird einer weiteren Destillationskolonne zugeführt, um das Isocyanat und Phosgen voneinander zu trennen und Phosgen in die Reaktion zurückzuführen. Nachteilig an dem beschriebenen Verfahren ist der hohe Druck von 10-50 bar, bei dem die Destillation betrieben werden muss, um die Gemische bei ökonomisch vorteilhaften Kühlmitteltemperaturen destillativ trennen zu können.

Die vorgenannten Druckschriften offenbaren keine konkrete Anweisung zum technischen Handeln, wie überschüssiges Phosgen auf möglichst wirtschaftliche Weise zurückgewonnen und in die Phosgenierungsreaktion zurückgeführt werden kann. Hierdurch verlieren die Verfahren an ökonomischer Attraktivität.

WO 2007/014 936 offenbart ein Verfahren zur Herstellung von Diisocyanaten durch Umsetzung von Diaminen mit stöchiometrisch überschüssigem Phosgen in der Gasphase, wobei das überschüssige Phosgen zumindest teilweise wieder in die Reaktion geführt wird und wobei der Phosgenstrom zum Reaktor vor der Vermischung mit dem Amin weniger als 15 Gew.-% Chlorwasserstoff enthält. Damit soll nach der Lehre dieser Schrift die Standzeit der Reaktoren verbessert werden, da Ausfällungen von Aminhydrochloriden verringert werden sollen. Nachteilig an solch hohen Gehalten an inertem Chlorwasserstoff Gas in dem Phosgengas ist, dass dieses über vergrößerte Kreislaufströme zu einer Erhöhung der Betriebskosten sowie zu großen Apparaten und damit zu hohen Anlagenbaukosten führt. Als Ausführungsform wird beschrieben, dass das überschüssige Phosgen und der entstandene Chlorwasserstoff zunächst von dem im wesentlichen gasförmigen Reaktionsgemisch abgetrennt wird, dann das überschüssige Phosgen zumindest teilweise wieder in die Reaktion zurückgeführt wird, wobei aus diesem rückgeführten Phosgen Chlorwasserstoff so abgetrennt wird, dass der Phosgenstrom vor der Vermischung mit dem Aminstrom weniger als 15 Gew.-% Chlorwasserstoff enthält. Über den Gehalt an Lösungsmittel im zurückgeführten Phosgenstrom werden keine Angaben gemacht. Die Schrift lehrt, dass die Trennung bevorzugt über eine Kombination von einer Destillation und einer Wäsche durchgeführt wird. Dabei wird das Phosgen aus dem Chlorwasserstoff-haltigen Strom mit einem Waschmittel ausgewaschen. Die Abtrennung des Phosgens und des Chlorwasserstoffes aus diesem beladenen Waschmedium erfolgt bevorzugt destillativ. Die Wäsche und die Destillation können nach Beschreibung bei Drücken von 1 bis 10 bar absolut betrieben werden. Weitere Erläuterungen zur Trennung von Phosgen aus dem beladenen Waschmittel offenbart die Schrift nicht.

Nach der Lehre von WO 2008/086 922 darf in einer Gasphasenphosgenierungsreaktion das Phosgen vor der Vermischung mit dem Amin nicht mehr als 1000 Gew.-ppm Chlor enthalten, da sonst aufgrund der hohen Temperaturen die Gefahr von Werkstoffversprödungen eintreten würde. Nach dieser Lehre bildet sich aufgrund der Spaltung von Phosgen bei hohen Temperaturen stets eine gewisse Menge Chlor, so dass eine Abtrennung dieses Chlors notwendig ist. Dazu offenbart die Schrift eine Ausführung, in der das Phosgen, Chlorwasserstoff und Chlor enthaltende Gasgemisch zunächst einer partiellen Kondensation (S. 18, Z.30) und Wäsche (S. 19, Z.18) unterworfen wird. Dabei wird eine flüssige Phase enthaltend Phosgen, Waschmedium, Chlorwasserstoff und Chlor erhalten. Diese wird dann in einer ersten Rektifikation (genannt c)) von den Leichtsiedern Chlor und Chlorwasserstoff befreit. In einem daran anschließenden Schritt werden in einer zweiten Rektifikation (genannt e)) (S. 20, Z. 26 bis S. 21 Z. 11) Phosgen und Waschmedium voneinander getrennt. Die Schrift offenbart zwei Ausführungsformen der zweiten Rektifikationskolonne: In der ersten verfügt die Rektifikationskolonne nur über einen Abtriebsteil, so dass das Kopfprodukt ohne Reinigung über trennwirksame Einbauten am Kopf abgezogen wird. Die Charakterisierung der Zusammensetzung des Sumpfproduktes ist nicht eindeutig; es wird in die Phosgenierreaktion zurückgeführt. Über die weitere Verwendung des Leichtsiederstroms eL wird keine Angabe gemacht.

In der bevorzugten zweiten Ausführungsform weist die zweite Rektifikation zusätzlich einen Verstärkerteil auf, der es bei entsprechendem Rücklaufverhältnis ermöglicht, dass der Kopfstrom im Wesentlichen aus reinem Phosgen besteht, das ohne weitere Aufreinigung in der Phosgenierung eingesetzt werden kann. Der Sumpfstrom besteht in dieser Ausführungsform mit Verstärkerteil im Wesentlichen aus reiner Waschflüssigkeit.

WO 2009/037 179 offenbart ein Verfahren zur Herstellung von Isocyanaten in der Gasphase, wobei das frisch erzeugte Phosgen ohne vorherige Kondensation der Gasphasenreaktion zugeführt wird. Indem auf die Apparate und Energie zur Phosgenkondensation, Flüssigphosgen-Zwischenlagerung und Phosgen-Verdampfung verzichtet werden kann, wird der Phosgen-Hold-up der Anlage verringert und die Energie zum Verdampfen des Phosgens eingespart (S. 5 Z. 32-42). Nachteilig an diesem Verfahren ist die fehlende Möglichkeit, im Frischphosgen enthaltene Begleitkomponenten abzutrennen, die ansonsten den aus dem Verfahren abgeführten Chlorwasserstoff-Strom verunreinigen.

Weiterhin beschreibt diese Schrift das Verfahren zur Abtrennung von Phosgen aus einem Gasgemisch mit Chlorwasserstoff und zur Rückführung des abgetrennten Phosgens zur Gasphasenphosgenierung durch eine kombinierte Wäsche und mehrstufige Destillation..

Die Schrift führt dazu aus, dass zunächst in einem ersten Schritt durch Wäsche des Phosgen-Chlorwasserstoff-Gasgemisches mit einer Waschflüssigkeit eine mit Phosgen und Chlorwasserstoff beladene Waschflüssigkeit erhalten wird. Daran schließt sich ein erster Destillationsschritt an, in dem der Chlorwasserstoff weitestgehend aus der Phosgen-haltigen Waschlösung entfernt und wieder in den vorgelagerten Waschschritt zurückgeführt wird, gefolgt von einem zweiten Destillationsschritt, in dem die zuvor erhaltende Waschlösung in gasförmiges Phosgen und weitestgehend phosgenfreie Waschflüssigkeit aufgetrennt wird. Das gasförmige Phosgen wird in die Gasphasenphosgenierung geleitet, während die Waschflüssigkeit erneut zur Wäsche des Phosgen-Chlorwasserstoff-Gasgemisches verwendet wird. Die Schrift offenbart nicht, wie die Phosgen-Waschflüssigkeit-Trennung apparativ ausgestaltet ist oder welche Reinheit des zurückgeführten Phosgenstroms erreicht werden.

Nach allgemeiner Lehre dieser Schrift ist folglich ein zweistufiges Destillationsverfahren, um gasförmiges Phosgen aus einem mit Phosgen beladenen Waschmedium für die Rückführung in die Phosgenierreaktion zurückzugewinnen, das Verfahren der Wahl. Der Wegfall der Frischphosgen-Kondensation und -bevorratung ist die einzige konkrete Maßnahme, die die Schrift zum Erzielen eines geringeren Phosgen-Hold-ups nennt. Obwohl als bevorzugte Ausführungsform eine aus zwei Destillationsschritten bestehende Prozessführung (und damit der Einsatz von Trennapparaten mit signifikantem Phosgen-Hold-up) vorgeschlagen werden, werden keine Detail-Angaben zur apparativen Ausgestaltung der zwei Destillationsschritte gemacht, insbesondere nicht zu einer den Phosgen-Hold-up minimierenden apparativen Ausgestaltung.

WO 2011/003 532 offenbart ein Verfahren zur Herstellung von Isocyanaten durch Umsetzung von primären Aminen mit Phosgen im stöchiometrischen Überschuss in der Gasphase, bei dem das überschüssige Phosgen anschließend zurückgewonnen und in die Umsetzung zurückgeführt wird. Die Rückgewinnung von Phosgen aus dem Phosgen und Chlorwasserstoff enthaltenden Gasgemisch erfolgt zweistufig. Im ersten Schritt (Chlorwasserstoff-Phosgen-Trennung) wird das Chlorwasserstoff und Phosgen enthaltende Gasgemisch, das den Reaktor verlässt, in einen gasförmigen im wesentlichen Chlorwasserstoff enthaltenden Strom und einen flüssigen, Phosgen enthaltenden Strom aufgetrennt, und in einem zweiten Schritt (Phosgengaserzeugung) der zuvor erhaltende flüssige Strom in einen gasförmigen, Phosgen enthaltenden Strom überführt, wobei der Druck im ersten Verfahrensschritt geringer ist als der Druck im zweiten Verfahrensschritt. Das Verfahren ist vorteilhaft, da es die Rückgewinnung von Phosgen aus der flüssigen, phosgenhaltigen Waschmittellösung in nur einem Schritt (Phosgengaserzeugung) ermöglicht. In einer bevorzugten Ausführungsform erfolgt die Phosgengaserzeugung in einer Destillationskolonne mit 2 - 45 theoretischen Trennstufen. Die Kolonne kann einen Abtriebsteil und / oder Verstärkerteil enthalten, bevorzugt enthält die Kolonne sowohl einen Abtriebs- als auch einen Verstärkerteil, mit bevorzugter Zufuhr des Eingangsstroms zwischen Abtriebs- und Verstärkerteil. Die Kolonne wird bevorzugt bei einer Sumpftemperatur von 140 - 220°C betrieben.

In einer bevorzugten Ausführungsform ist die Kolonne mit einem Kopfkondensator versehen. Der Kopfkondensator wird bevorzugt bei einer Kühlmitteleintrittstemperatur von -25 bis 0 °C betrieben. Das durch den Kopfkondensator erzeugte Kondensat kann ganz oder teilweise auf die Kolonne zurückgeführt und / oder entnommen werden, bevorzugt wird das Kondensat vollständig auf die Kolonne zurückgeführt.

Nachteilig an der bevorzugten Ausführungsform ist die große Dimension der Kolonne zur Phosgengaserzeugung, sowie der teure Einsatz von Kälte zur Erzeugung eines Rücklaufstroms.

In einer alternativen Ausführungsform wird die Phosgengaserzeugung so durchgeführt, dass der Phosgen enthaltende flüssige Strom aus der Chlorwasserstoff-Phosgen-Trennung durch partielle Verdampfung in einen Phosgen und ggf. Inerte enthaltenden gasförmigen Strom und in einen flüssigen Strom aufgetrennt wird. Dazu wird der aus der Chlorwasserstoff-Phosgen-Trennung erhaltene flüssige Strom in einen Verdampfer geführt, dessen Sumpftemperatur bevorzugt 100 - 220°C beträgt. Diese Ausführungsform reduziert im Vergleich zur vorgenannten bevorzugten Ausführungsform die Apparate- und Kälteenergiekosten, sowie den flüssigen Phosgen-Hold-up, ermöglicht jedoch nur geringe Reinheiten des erzeugten gasförmigen Phosgenstroms sowie des aus dem Verdampfersumpf flüssig ausgeschleusten, vorwiegend Waschmittel enthaltenden Stroms.

Darüber hinaus lehrt die Schrift, dass zur Optimierung des Energieverbrauchs der Phosgen enthaltende flüssige Strom aus der Chlorwasserstoff-Phosgen-Trennung bevorzugt indirekt in die Phosgengaserzeugung geleitet wird, d. h. durch Wärmeaustausch mit anderen Prozessströmen um insbesondere bevorzugt 5 - 175°C erwärmt der Phosgengaserzeugung zugeführt wird.

Gemäß Beispiel 5 in WO 2011/003 532 ist die Phosgengaserzeugung in Form einer Strippkolonne, also ohne Verstärkungsteil, ausgeführt. (Als *Verstärkungsteil* bezeichnet man in der Destillationstechnik den durch Einbauten trennwirksamen Teil oberhalb des Zulaufs einer Destillationskolonne. Der Verstärkungsteil erhöht die Reinheit des Kopfproduktes. Bei einer Strippkolonne wird der zu destillierende Strom am Kopf der Destillationskolonne aufgegeben, sodass eine Strippkolonne nicht über einen Verstärkungsteil verfügt.) Die aus der Chlorwasserstoff-Phosgen-Trennung erhaltene Phosgenlösung wird mit einer Temperatur unterhalb von 10 °C direkt, also ohne Vorverdampfung, auf den Kopf der Strippkolonne gepumpt. Am Kopf der Strippkolonne wird gasförmiges Phosgen entnommen, das ca. 0,2 Gew% Lösemittel enthält. Nachteilig an dieser Ausführungsform ist jedoch, dass aufgrund der niedrigen Kopftemperatur der Desorptionskolonne keine energiesparende Wärmeintegration durch Vorwärmung und partielle Vorverdampfung realisiert werden kann. Des Weiteren kann die Phosgenlösung aufgrund von variierten Prozessparametern (u. a. Phosgenüberschuss in der Reaktion, Reaktoraustrittstemperatur, Lösungsmittelmenge zur Quenche, Lösungsmittelmenge zur Phosgenabsorption) und aufgrund von Prozessschwankungen unterschiedliche Zusammensetzungen und Temperatur aufweisen. Dies führt bei direkter Aufgabe auf den Kopf der Strippkolonne unweigerlich zu einer geänderten und ggf. schwankenden Zusammensetzung und Temperatur des Phosgenrückführstroms zur Reaktion, da ein stabilisierend wirkender, regelbarer Kopfkondensator in dieser Ausführungsform nicht zur Verfügung steht. Schwankende Bedingungen des Phosgenrückführstroms können zu geringerer Energieeffzienz, verringerter Reaktionsausbeute, erhöhtem Betriebsaufwand und reduzierter Verfügbarkeit führen.

In WO 2011/003 532 wird die Möglichkeit erwähnt, dass Chlorwasserstoff-Phosgen-Gasgemische aus mehreren Reaktionsstraßen in einer einzigen Chlorwasserstoff-Phosgen-Trennung behandelt werden. Es wird jedoch nicht offenbart, wie der für den parallelen Betrieb mehrerer Reaktoren essentielle, ausreichend stabile Kolonnendruck auch bei schnellen Betriebspunktänderungen gewährleistet werden kann. Da Gasphasenreaktoren einen engen günstigen Lastbereich aufweisen, ist die schnelle Betriebspunktänderung z.B. beim An- und Abfahren einzelner Reaktoren vorteilhaft.

Aus dem Stand der Technik sind also eine Reihe von Verfahrensweisen bekannt, wie Gemische aus Chlorwasserstoff und Phosgen sowie ggf. Lösungsmittel aufgetrennt werden können, um eine Rückführung des Phosgens in die Reaktion zu ermöglichen. Der Stand der Technik befasst sich jedoch nicht eingehend mit der Problematik, die Phosgenrückführung auch unter erschwerten Bedingungen regelungstechnisch beherrschbar zu machen. Solche erschwerten Bedingungen können beispielsweise unerwartete Prozessschwankungen beispielsweise ausgelöst durch Schwankungen oder kurzfristige Unterbrechungen in einzelnen Teilanlagen, z. B. der Frischphosgenerzeugung, sein. Aber auch geplante Änderungen am Prozess können zu regelungstechnischen Herausforderungen werden. Dies ist insbesondere dann der Fall, wenn eine Gasphasenphosgenierung in mehreren parallel geschalteten Reaktoren, *deren gasförmige Reaktionsprodukte in einem gemeinsamen Aufarbeitungsteil aufgearbeitet werden,* durchgeführt werden soll.

Der parallele Betrieb mehrerer gleichgestalteter Fertigungseinrichtungen ist der großindustriellen Produktion chemischer Produkte nichts Ungewöhnliches. Die Aufteilung einer angestrebten Gesamtproduktionskapazität auf mehrere parallel betriebene Fertigungseinrichtungen (Produktionsstrecken oder -straßen) ist u. a. dann empfehlenswert, wenn
- Die Apparatebauweise eine kleinere maximale Produktionskapazität erlaubt als die gewünschte Gesamtkapazität der Produktionsanlage (vgl. "Rules of Thumb for Chemical Engineers, 4th Ed., 2005, S. 242, Kapitel "Process Evaluation - What Size should a plant be? ")
- Teilprozesse oder Apparate z. B. wegen Verschmutzungsneigung zu Reinigungszwecken gelegentlich abgeschaltet werden müssen, ohne dass dazu die gesamte Produktion abgeschaltet werden soll (Ullmann's Encyclopedia of Industrial Chemistry, Wiley-VCH, Weinheim, 2012, Kapitel "Chemical Plant Design and Construction", Band 8, S. 267)
- Teilprozesse oder Apparate ein höheres Ausfallrisiko aufweisen, als es für die Gesamtanlage in Kauf genommen werden soll (Ullmann's Encyclopedia of Industrial Chemistry, Wiley-VCH, Weinheim, 2012, Kapitel "Chemical Plant Design and Construction", Band 8, S. 273)
- Teilprozesse oder Apparate einen eingeschränkteren möglichen Lastbereich aufweisen, als er für die Gesamtanlage ermöglicht werden soll

EP-A-570 799 beschreibt, dass Gasphasenreaktoren für die Phosgenierung von Aminen nur einen engen günstigen Lastbereich aufweisen, und dass Feststoffbildung in den Reaktoren auftreten kann, die Produktionsunterbrechungen zu Reinigungszwecken notwendig machen kann. U. a. aus diesen Gründen kann die Installation von mehreren parallel betreibbaren Reaktionsstrecken zur Phosgenierung vorteilhaft sein. Zur Reduzierung der Apparatekosten kann es dagegen vorteilhaft sein, vor- und/oder nachgeschaltete Prozessschritte (z. B. Phosgenerzeugung, Aminverdampfung, Chlorwasserstoff-Phosgen-Trennung) trotzdem für alle Reaktionsstrecken gemeinsam auszugestalten.

EP-A-2 196 455 beschreibt u. a. eine Ausführungsform eines Herstellverfahrens für Isocyanate, bei der das Rohprodukt zweier Reaktionsstrecken einer gemeinsamen Reaktionsabbruchszone (Quenche) zugeführt wird.

Wird eine gemeinsame Phosgengaserzeugung betrieben, die den zurückgewonnenen Phosgenstrom gasförmig auf mehrere Reaktionsstrecken zurückführt, sind die Phosgengaserzeugung und die Reaktoren über einen Gasraum miteinander verbunden, so dass sich eine Änderung im Prozessdruck in einem Apparat auf die verbundenen Apparate auswirkt. Dies gilt insbesondere, wenn sich keine aktiv regelbaren Druckerhöhungs- oder Druckreduzierungselemente in der Verbindung zwischen Phosgengaserzeugung und Reaktoren befinden. Vor allem plötzliche Änderungen von Prozessparametern (z. B. Sicherheitsabschaltung oder Laständerung in einer Reaktionsstraße) führen zu unerwünschten Instabilitäten in der Gesamtanlage, ggf. bis hin zur Notwendigkeit, die Gesamtanlage außer Betrieb nehmen zu müssen. Ein wesentlicher Parameter für den störungsfreien und stabilen Betrieb der Reaktion ist die Sicherstellung eines konstanten Phosgenüberschusses in der Phosgenierungsreaktion. Um einen konstanten Strom von gasförmigem Phosgen sicherzustellen, ist ein konstanter Versorgungsdruck der Phosgengas-Quelle, also z. B. einer Destillationskolonne, essentiell. Damit kommt der Druckregelung dieser Destillationskolonne eine wesentliche Bedeutung für die Gesamtprozessstabilität zu.

Um die Apparatekosten zu senken und dem zunehmenden sicherheitstechnischen Wunsch Rechnung zu tragen, den Phosgen-Hold-up zu minimieren, wäre es vorteilhaft, auf den Verstärkerteil und möglichst auch auf den Kopf-Kondensator der zur Phosgengaserzeugung eingesetzten Destillationskolonne verzichten zu können. Der Wegfall des Kopf-Kondensators erschwert jedoch die Druckregelung. Darüber hinaus wird im Stand der Technik kein Verfahren offenbart, das den Wegfall des Verstärkerteils mit einer energieeffizienten Vorwärmung / Vorverdampfung des Phosgengaserzeugungs-Zulaufs vereinen würde.

Im Stand der Technik wird zwar erwähnt, dass der Gasphasenreaktion ein inerter Stoff, z. B. Lösungsmittel, zugeführt werden kann, wobei der Bereich der beschriebenen möglichen Lösungsmittelgehalte im Phosgenrückführstrom bis 10 Massen-% reicht. Jedoch wird in allen offenbarten konkreten Ausführungsbeispielen, die die Rückgewinnung und Rückführung von überschüssig eingesetztem Phosgen zurück in die Gasphasenreaktion beschreiben, offenbar *in der Praxis* trotzdem *ein möglichst niedriger Lösungsmittelgehalt* im Phosgenstrom angestrebt (siehe z.B. die weiter oben ausführlich diskutierte Druckschrift WO 2011/003 532: gemäß dieser werden nur wenige ppm Lösungsmittel zugelassen, um den Energieeinsatz zu minimieren; in Beispiel 5 wird eine Lösungsmittel-Konzentration von 0,2 % genannt, was der maximale *in einem konkreten Ausführungsbeispiel* offenbarte Wert ist). Dies geschieht, indem entweder die Phosgen-Lösungsmittel-Trennkolonne mit einem Verstärkerteil ausgerüstet ist und mit einem Rücklauf betrieben wird, oder indem ein niedriges Temperaturniveau am Kopf der Kolonne eingehalten wird (in WO 2011/003 532 wird eine Temperatur des Zulauf auf den Kolonnenkopf von maximal 10 °C genannt).

Im Stand der Technik wird demnach keine Anleitung gegeben, wie sich die Vorteile von
a. einer Phosgengaserzeugung ohne Verstärkerteil (geringere Apparatekosten, geringerer Phosgen-Hold-up, geringere Kondensations-Energie-Kosten) und
b. einer energieeffizienten Wärmeintegration zur Zulauf-Vorwärmung und/oder -verdampfung
miteinander kombinieren lassen.

Es bestand daher ein Bedarf an einem apparativ möglichst einfachen und energetisch günstigen Verfahren zur Herstellung von Isocyanaten, das den Phosgen-Hold-up möglichst gering hält, ohne dadurch die Rückführung des überschüssigen Phosgens in die Reaktion insbesondere unter regelungstechnisch herausfordernden Bedingungen (wie z. B. dem Einsatz mehrerer parallel betriebener Reaktionsstrecken mit gemeinsamer Aufarbeitung) zu erschweren.

Diesem Bedarf Rechnung tragend ist ein Gegenstand der vorliegenden Erfindung ein kontinuierliches Verfahren zur Herstellung eines Isocyanats durch Phosgenierung des korrespondierenden primären Amins, umfassend die Schritte:
(i) Umsetzung des primären Amins mit einem Überschuss an Phosgen in der Gasphase;
(ii) Behandlung des in (i) erhaltenen Verfahrensprodukts mit einem Lösungsmittel bei einer Temperatur, die unterhalb der Siedetemperatur des Isocyanats und oberhalb der Zersetzungstemperatur des korrespondierenden Carbaminsäurechlorids liegt, unter Erhalt eines gasförmigen, Chlorwasserstoff sowie nicht umgesetztes Phosgen enthaltenden Stroms (ii-1) und eines flüssigen, Lösungsmittel sowie Isocyanat enthaltenden Stroms (ii-2);
(iii) Trennung des in Strom (ii-1) enthaltenen Chlorwasserstoffs und Phosgens unter Erhalt eines flüssigen Phosgen-haltigen Stroms (iii-1) und eines gasförmigen Chlorwasserstoff-haltigen Stroms (iii-2);
(iv) Partielle Verdampfung des flüssigen Phosgen-haltigen Stroms (iii-1) unter Erhalt eines zweiphasigen Verfahrensproduktes;
(v) Aufgabe des zweiphasigen Verfahrensproduktes aus Schritt (iv) am Kopf einer Destillationskolonne, welcher ein gasförmiger Phosgen-haltiger Strom über Kopf entnommen wird;
(vi) Rückführung des gasförmigen Phosgen-haltigen Stroms aus Schritt (v) in Schritt (i) (Rezyklierung von "Rückphosgen");
(vii) Aufarbeitung des in Schritt (ii) erhaltenen flüssigen, Lösungsmittel sowie Isocyanat enthaltenden Stroms (ii-2) zur Gewinnung des gewünschten Isocyanats.

Die erfindungsgemäße Vorgehensweise, den Zulauf der Destillationskolonne aus Schritt (v) am Kopf dieser Kolonne einzurichten, d. h., auf einen Verstärkungsteil zu verzichten, führt im Vergleich zu der im Stand der Technik üblichen Ausführungsform mit Verstärkungsteil zu erhöhten Lösungsmittelgehalten im gasförmigen Phosgen-haltigen Kopfprodukt dieser Kolonne. Überraschend wurde jedoch gefunden, dass die damit zwangsläufig verbundene Erhöhung des Volumenstroms (welche an und für sich nicht erstrebenswert ist), hinter den vielfältigen anderen Vorteilen des erfindungsgemäßen Verfahrens (apparative Vereinfachung, geringerer Phosgen-Hold-up, Betriebsstabilität, verbesserte Möglichkeit der Energieintegration) zurücktritt und die Vorteile insgesamt deutlich überwiegen.

Ferner ermöglicht es die erfindungsgemäße Vorgehensweise, den flüssigen Phosgen-haltigen Strom (iii-1) in Schritt (iv) nur partiell zu verdampfen und in Schritt (v) ein zweiphasiges Verfahrensprodukt in die Destillationskolonne einzuspeisen, den in Schritt (iv) eingesetzten Vorverdampfer als Regelungseinrichtung zu verwenden, wodurch der bisher übliche Kopfkondensator dieser Destillationskolonne, der im Stand der Technik auch Regelungsaufgaben übernimmt, prinzipiell verzichtbar wird und bevorzugt im erfindungsgemäßen Verfahren auch nicht eingesetzt wird. Über die Betriebsweise des Vorverdampfers lässt sich der Lösungsmittelgehalt im gasförmigen Phosgen-haltigen Kopfprodukt gezielt einstellen (z. B. auf 5,0 Massen-% bei einer Temperatur des zweiphasigen Verfahrensprodukts bei Einspeisung in die Destillationskolonne von 100 °C), wie weiter unten noch näher ausgeführt wird. (Alle in dieser Schrift angegebenen Zusammensetzungen beziehen sich die Masse der jeweiligen Komponente in Bezug auf die Masse des jeweiligen Gesamtstromes, sofern nicht in den entsprechenden Passagen eine andere Definition vorgenommen wird.)

Das erfindungsgemäße Zusammenspiel von apparativer Ausgestaltung (kein Verstärkungsteil in der Destillationskolonne aus Schritt (v); in einer bevorzugten Ausführungsform Verzicht auf den Kopfkondensator) und Betriebsweise (nur partielle Verdampfung im Vorverdampfer von Schritt (iv)) ermöglicht es, den Druck in der Erzeugung gasförmigen Rückführphosgens (d. h. den Druck in der Destillationskolonne aus Schritt (v)) so stabil zu regeln, dass auch bei plötzlichen, erheblichen Betriebspunktänderungen der Phosgenmengenstrom zu allen in Schritt (i) eingesetzten Reaktoren nur geringe Abweichungen vom Sollwert zeigt. Diese Qualität der Druckregelung lässt sich, wie weiter unten noch im Detail ausgeführt wird, in einer bevorzugten Ausführungsform dadurch erreichen, dass ein (mindestens teilweise) dampfförmiger, phosgenhaltiger Zulaufstrom zur Stufe der Erzeugung gasförmigen Rückführphosgens als Stellgröße der Druckregelung verwendet wird.

Nachstehend werden verschiedene Ausführungsformen der Erfindung näher beschrieben. Dabei sind alle Ausführungsformen beliebig miteinander kombinierbar, sofern sich für den Fachmann aus dem Kontext nicht das Gegenteil ergibt.

**Schritt** (i) der Erfindung, die eigentliche Gasphasenphosgenierung, kann grundsätzlich nach einem aus dem Stand der Technik beschriebenen Verfahren durchgeführt werden, wie sie beispielsweise in EP-A-570 799 und WO-A-2007/014936 beschrieben werden.

Dabei können sowohl aliphatische als auch aromatische Mono- und Poly-Amine eingesetzt werden. Bevorzugt werden aromatische Amine eingesetzt, insbesondere bevorzugt aromatische Diamine, die ohne nennenswerte Zersetzung in die Gasphase überführt werden können.

Beispiele für bevorzugte aromatische Amine sind Toluylendiamin (TDA), insbesondere 2,4-TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus.

Weiterhin sind besonders Amine, insbesondere Diamine, auf Basis von aliphatischen oder cycloaliphatischen Kohlenwasserstoffen mit 2 bis 18 Kohlenstoffatomen geeignet. Besonders gut geeignete Amine sind 1,6-Diamino-hexan, l-Amino-3,3,5-trimethyl-5-aminomethylcyclohexan (IPDA) und 4,4'-Diaminodicyclohexylamin.

Die Ausgangsamine werden vor der Durchführung des erfindungsgemäßen Verfahrens in der Regel verdampft und auf 200°C bis 600 °C, bevorzugt 200°C bis 500 °C, besonders bevorzugt 250°C bis 450 °C erhitzt und gegebenenfalls verdünnt mit einem Inertgas wie N₂, He, Ar oder mit den Dämpfen eines inerten Lösungsmittels, z. B. aromatischen Kohlenwasserstoffen ggf. mit Halogensubstitution, wie z. B. Chlorbenzol oder o-Dichlorbenzol, dem Reaktionsraum zugeführt.

Die Verdampfung der Ausgangsamine kann in allen bekannten Verdampfungsapparaturen erfolgen, bevorzugt werden Verdampfungssysteme, bei denen ein kleiner Arbeitsinhalt mit einer hohen Umwälzleistung über einen Fallfilmverdampfer geführt wird, wobei zur Minimierung der thermischen Belastung der Ausgangsamine der Verdampfungsvorgang - wie oben ausgeführtggf. unter Zuspeisung von Inertgas und / oder Dämpfen eines inerten Lösungsmittels unterstützt werden kann. Alternativ kann die Verdampfung auch in speziellen Verdampfungsapparaturen erfolgen mit sehr kurzen Verweilzeiten wie sie zum Beispiel in EP-A-1 754 698 beschrieben sind.

Bei dem erfindungsgemäßen Verfahren wird Phosgen im Überschuss bezüglich der umzusetzenden Amingruppen eingesetzt. Bevorzugt liegt ein molares Verhältnis von Phosgen zu Amingruppen von 1,1 bis 20, bevorzugt von 1,2 bis 5, vor. Auch das Phosgen wird auf Temperaturen von 200°C bis 600 °C erhitzt und gegebenenfalls verdünnt mit einem Inertgas wie N₂, He, Ar oder mit den Dämpfen eines inerten Lösungsmittels, z. B. aromatischen Kohlenwasserstoffen ohne oder mit Halogensubstitution, wie z. B. Chlorbenzol oder o-Dichlorbenzol, dem Reaktionsraum zugeführt.

Das erfindungsgemäße Verfahren wird in Schritt (i) bevorzugt so durchgeführt, dass die getrennt aufgeheizten Reaktionspartner über zumindest eine Mischeinrichtung in wenigstens einen Reaktionsraum eingeführt, vermischt und unter Beachtung geeigneter Reaktionszeiten unter bevorzugt adiabater Reaktionsführung umgesetzt werden. Anschließend wird durch Abkühlung des Gasstromes das Isocyanat kondensiert, wobei die Abkühlung bis zu einer Temperatur oberhalb der Zersetzungstemperatur des entsprechenden Carbaminsäurechlorids, also beispielsweise Toluylendiaminsäurechlorid im Falle von TDA, erfolgt.

Die notwendige Verweilzeit zur Reaktion der Amingruppen mit dem Phosgen zu Isocyanat liegt zwischen 0,05 und 15 Sekunden, in Abhängigkeit von der Art des eingesetzten Amins, der Starttemperatur, der adiabaten Temperaturerhöhung im Reaktionsraum, dem molaren Verhältnis von eingesetztem Amin und Phosgen, einer etwaigen Verdünnung der Reaktionspartner mit Inertgasen sowie dem gewählten Reaktionsdruck.

Besonders bevorzugt kommen in Schritt (i) Reaktoren mit im Wesentlichen rotationssymmetrischen Reaktionsräumen zum Einsatz, bei denen die gasförmigen Edukte, ggf. verdünnt mit Inerten, dem zumindest einen Mischraum nach dem Strahlmischerprinzip (Chemie-Ing. Techn. 44 (1972) S. 1055, Abb.10) zugeführt werden.

Bevorzugt weisen weder der Reaktionsraum noch etwaige Mischaggregate oder Mischräume Heizflächen auf, die zu einer thermischen Belastung mit der Folge von Folgereaktionen wie Isocyanurat- oder Carbodiimidbildung Anlass geben können, oder Kühlflächen auf, die Anlass einer Kondensation mit der Folge von Ablagerungen geben können. Die Komponenten werden so, abgesehen von etwaigen Abstrahlungs- und Ableitungsverlusten, bevorzugt adiabat umgesetzt, dabei wird die adiabate Temperaturerhöhung im Mischaggregat und Reaktor bzw. Reaktor allein über die Temperaturen, Zusammensetzungen und relativen Dosierungen der Eduktströme sowie der Verweilzeit in den Mischaggregaten und den Reaktoren eingestellt. Möglich ist bei dem erfindungsgemäßen Verfahren auch eine nicht adiabatische Umsetzung der Komponenten.

Nach der erfolgten Phosgenierungsreaktion im Reaktionsraum werden das gasförmige Reaktionsgemisch, das das gebildete Isocyanat, Phosgen und Chlorwasserstoff umfasst, in **Schritt** (ii) durch Behandlung mit einem Lösungsmittel von dem gebildeten Isocyanat befreit und evtl. noch ablaufende Reaktionen abgebrochen ("Quenche").

Dies kann beispielsweise dadurch erfolgen, indem das den Reaktionsraum kontinuierlich verlassende Verfahrensprodukt nach Verlassen des Reaktionsraumes einer Kondensation in einem inerten Lösungsmittel unterzogen wird, wie sie bereits für andere Gasphasenphosgenierungen empfohlen wurde (vgl. EP-A-0 749 958).

Bevorzugt erfolgt die Kondensation jedoch dadurch, dass das aus dem Reaktionsraum austretende Verfahrensprodukt in eine Reaktionsabbruchszone geführt wird, in die ein oder mehrere geeignete Lösungsmittelströme ("Quench-Flüssigkeiten") eingesprüht werden. Dadurch kann, wie in EP-A-1 403 248 beschrieben, eine rasche Abkühlung der Gasgemische ohne den Einsatz kalter Flächen durchgeführt werden.

In jedem Fall erfolgt die Behandlung mit Lösungsmittel bei einer Temperatur, die unterhalb der Siedetemperatur des Isocyanats und oberhalb der Zersetzungstemperatur des korrespondierenden Carbaminsäurechlorids liegt, sodass ein gasförmiger, Chlorwasserstoff sowie nicht umgesetztes Phosgen enthaltender Strom (ii-1) und ein flüssiger, Lösungsmittel sowie Isocyanat enthaltender Strom (ii-2) erhalten werden. Zur selektiven Gewinnung des Isocyanats aus dem gasförmigen Reaktionsgemisch besonders gut geeignet sind bei einer Temperatur von 80 °C bis 200 °C, vorzugsweise 80 °C bis 180 °C gehaltene Lösungsmittel wie beispielsweise Chlorbenzol und / oder Dichlorbenzol, oder in diesen Temperaturbereichen gehaltenes Isocyanat oder Mischungen des Isocyanats mit Chlorbenzol und / oder Dichlorbenzol. Es ist für den Fachmann aufgrund der physikalischen Daten bei gegebener Temperatur, Druck und Zusammensetzung einfach vorhersagbar, welcher Massenanteil des Isocyanats in der Quenche kondensiert bzw. diese nicht kondensiert durchläuft. Ebenso ist es einfach vorherzusagen, welcher Massenanteil des überschüssigen Phosgens, Chlorwasserstoffs und ggf. als Verdünnungsmittel verwendeten Inertgases die Quenche unkondensiert durchläuft bzw. sich in der Quencheflüssigkeit löst.

Das die Kondensations- bzw. Quenchestufe verlassende Gasgemisch (ii-1) wird bevorzugt in einer nachgeschalteten Gaswäsche mit einer geeigneten Waschflüssigkeit von restlichem Isocyanat befreit. Die Reindarstellung des Isocyanats erfolgt bevorzugt anschließend durch destillative Aufarbeitung des Stroms (ii-2), ggf. nachdem dieser mit weiterem Isocyanat aus der Gaswäsche vereint wurde.

In **Schritt** (iii) erfolgt die Trennung des in Strom (ii-1) enthaltenen Chlorwasserstoffs und Phosgens unter Erhalt eines flüssigen Phosgen-haltigen Stroms (iii-1) und eines gasförmigen Chlorwasserstoff-haltigen Stroms (iii-2).

Das in die Auftrennung in Schritt (iii) eintretende Gasgemisch enthält in der Regel 1 bis 50 Massen-% HCl, bevorzugt 3 bis 40 Massen-% HCl, insbesondere bevorzugt 5 bis 35 Massen-% HCl und ganz besonders bevorzugt 7,5 bis 30 Massen-% HCl, bezogen auf die Masse des Gasgemisches. Dieses Gasgemisch enthält in der Regel 5 bis 90 Massen-% Phosgen, bevorzugt 15 bis 85 Massen-% Phosgen, insbesondere bevorzugt 25 bis 80 Massen-% Phosgen und ganz besonders bevorzugt 40 bis 75 Massen-% Phosgen, bezogen auf die Masse des Gasgemisches. Der Gehalt an Lösemittel in dem Gasgemisch beträgt in der Regel 0,01 bis 60 Massen-%, bevorzugt 0,05 bis 40 Massen-% und insbesondere bevorzugt 0,1 bis 10 Massen-%, bezogen auf die Masse des Gasgemisches. Das Lösemittel kann dampfförmig oder auch flüssig vorliegen. Das Gasgemisch kann zudem inerte Gase in Summe von in der Regel 0 bis 10 Massen-%, bevorzugt 0,0001 bis 8 Massen-% und insbesondere bevorzugt 0,001 bis 5 Massen-%, bezogen auf die Massen des Gasgemisches, enthalten. Das Gasgemisch kann in der Regel 0 bis 10 Massen-%, bevorzugt 0,001 bis 7,5 Massen-% und insbesondere bevorzugt 0,05 bis 5 Massen-%, bezogen auf die Masse des Gasgemisches, an Reaktionsprodukt enthalten.

Die erfindungsgemäße Trennung in Schritt (iii) kann durch verschiedene Ausführungsformen erfolgen, wie sie beispielsweise in WO 2011/003532 (S. 11, Z. 31 bis S. 19 Z. 11) ausführlich beschrieben sind und auch im erfindungsgemäßen Verfahren eingesetzt werden können. So ist eine **Absorption in einem Lösungsmittel** ebenso geeignet wie eine **partielle Kondensation mit einer anschließenden Wäsche** oder eine **vollständige oder partielle Kondensation mit einer anschließenden Destillation oder Strippung.**

Eine besonders bevorzugte Ausführungsform für diesen Verfahrensschritt ist eine **Absorption in einem Lösungsmittel.** Insbesondere bevorzugt wird die Absorption dem Lösungsmittel durchgeführt, das auch für die Quenche (Schritt (ii)) verwendet wird.

In einer besonders bevorzugten Ausführungsform erfolgt die Absorption in einer Sequenz aus zumindest zwei Absorptionsschritten gegebenenfalls in Kombination mit partiellen Kondensationsstufen, wobei zumindest ein Absorptionsschritt isotherm und zumindest ein Absorptionsschritt adiabatisch geführt wird. Ganz besonders bevorzugt wird der erste Absorptionsschritt isotherm, der folgende Absorptionsschritt adiabatisch geführt. In einer insbesondere bevorzugten Ausführungsform wird für den adiabatischen und isothermen Absorptionsschritt jeweils das gleiche Lösungsmittel verwendet, das in Schritt (ii) eingesetzt wurde. Es ist weiterhin bevorzugt, das die letzte Absorptionsstufe verlassende Gas durch Abkühlen mittels eines Wärmeaustauschers von evtl. verbleibenden Spuren an Phosgen und Lösungsmittel durch Kondensation weiter zu reinigen. In einer bevorzugten Ausführungsform erfolgt die isotherme und folgende adiabatische Absorption in einem einzigen Apparat (= einer einzigen Absorptionskolonne), besonders bevorzugt erfolgt auch die Abkühlung des die Absorption verlassenden Gasstromes in dem gleichen Apparat. Dieses hat den Vorteil, dass dadurch die Anzahl der Flansche verringert wird, was zu einer Erhöhung der Sicherheit bei der Handhabung von Phosgen beiträgt. Es hat den weiteren Vorteil der Energieeinsparung, da durch die kompakte Bauweise in einem Apparat Energieverluste in den verbindenden Rohrleitungen minimiert werden.

In einer weiteren Ausführungsform wird Schritt (iii) durch **partielle Kondensation mit anschließender Wäsche** durchgeführt. In dieser besonderen Ausführungsform wird das Gasgemisch zunächst partiell kondensiert. Der verbleibende Gasstrom wird von unten in eine Absorptionskolonne eingeleitet und im Gegenstrom mit dem Lösungsmittel gewaschen. Dabei erfolgt die Abführung der Absorptionswärme durch externe Wärmeaustauscher. Dazu kann bevorzugt an verschiedenen Stellen der Absorptionskolonne die Flüssigkeit vollständig oder teilweise, bevorzugt vollständig, entnommen werden und durch einen externen Kühler abgekühlt werden.

Eine weitere mögliche Ausführungsform für die Durchführung von Schritt (iii) ist die **partielle oder vollständige Kondensation** von Phosgen, anschließend eine **Destillation oder Strippung** in einer Kolonne zur Entfernung des gelösten HCl aus dem Sumpfprodukt Phosgen und anschließend eine **Wäsche** des im ersten Schritt erhaltenden Kopfproduktes HCl mit einem Lösungsmittel zur Absorption des nach der Kondensation im Gasstrom verbliebenen Phosgens. Der im Sumpf der Destillation oder Strippung erhaltende flüssige Strom hat nur noch eine geringe Beladung mit gelöster HCl und / oder inerte Gase und kann in Schritt (iv) geleitet werden.

Die vorstehend beschriebenen Verfahrensalternativen zur Durchführung des Schrittes (iii) liefern alle einen gasförmigen Strom (iii-2) und einen flüssigen Strom (iii-1). Der HCl enthaltende Gasstrom (iii-2) hat eine ausreichende Reinheit und kann im Allgemeinen ohne weitere Reinigung weiter verarbeitet werden.

In einer Ausführungsform der vorliegenden Erfindung wird das zur Ergänzung des in Schritt (i) verbrauchten Phosgens zuzuführende Frischphosgen in Schritt (iii) zugegeben und wird damit Bestandteil des in diesem Schritt erhaltenen flüssigen Phosgen-haltigen Stroms (iii-1). Hierzu gibt es verschiedene Möglichkeiten. So kann Frischphosgen beispielsweise in der Ausgestaltung von Schritt (iii), in welcher dieser Schritt die Absorption in einem Lösungsmittel umfasst, ggf. nach Verflüssigung, in den Sumpf der entsprechenden Absorptionskolonne gegeben werden.

Der Schritt (iii) verlassende Gasstrom (iii-2) enthält im Wesentlichen HCl sowie gegebenenfalls Spuren von Phosgen. Neben HCl kann der Strom zudem noch inerte Gase und / oder Lösungsmittel sowie Spuren an Reaktionsnebenprodukten enthalten. Der Strom enthält 80 bis 100 Massen-%, bevorzugt 90 bis 100 Massen-% und insbesondere bevorzugt 95 bis 100 Massen-% HCl, bezogen auf die Masse des Gasstroms (iii-2). Dieser Gasstrom enthält maximal 0,8 Massen-% Phosgen, bevorzugt maximal 0,4 Massen-% und insbesondere bevorzugt maximal 0,2 Massen-% Phosgen, bezogen auf die Masse des Gasstroms (iii-2). Zur energetischen Optimierung kann es bevorzugt sein, mindestens 1 Massen-ppm Phosgen, bevorzugt mindestens 5 Massen-ppm Phosgen bezogen auf die Masse des Gasstroms (iii-2) zuzulassen, wobei in beiden Fällen die zuvor genannten Höchstgrenzen für den Phosgengehalt gelten. Der Schritt (iii) verlassende Gasstrom (iii-2) steht in der Regel am Austritt aus dem Verfahrensschritt unter einem Druck von 1,00 bis 4,00 bar absolut, bevorzugt von 1,01 bis 3,00 bar absolut und besonders bevorzugt 1,02 bis 2,00 bar absolut. Der aus Schritt (iii) erhaltene Gasstrom hat in der Regel am Austritt aus dem Verfahrensschritt eine Temperatur von -40 bis 30 °C, bevorzugt -20 bis 20 °C und besonders bevorzugt -15 bis 10 °C. Als Austritt aus dem Verfahrensschritt wird der Gasaustrittstutzen des letzten zu diesem Verfahrensschritt gehörigen Apparates verstanden.

Unabhängig von der genauen Ausgestaltung von Schritt (iii) enthält der diesen Schritt verlassende flüssige Phosgen enthaltende Strom (iii-1) neben Phosgen noch Lösungsmittel (nicht vollständig abgetrenntes Lösungsmittel aus Schritt (ii) und ggf. das Absorptions-Lösungsmittel aus Schritt (iii)). Gegebenenfalls können auch noch gelöste HCl und / oder gelöste Inertstoffe sowie gegebenenfalls gelöste Reaktionsnebenprodukte enthalten sein. Der Strom (iii-1) enthält 30 bis 90 Massen-%, bevorzugt 35 bis 85 Massen-% und besonders bevorzugt 40 bis 70 Massen-% Phosgen, bezogen auf die Masse des flüssigen Phosgen enthaltenden Stroms (iii-1). Zudem enthält dieser Strom 10 bis 70 Massen-%, bevorzugt 15 bis 65 Massen-% und insbesondere bevorzugt 30 bis 60 Massen-% an Lösungsmittel, bezogen auf die Masse des flüssigen Phosgen enthaltenden Stroms (iii-1). Weiterhin kann dieser flüssige Strom (iii-1) 0 bis 5 Massen-%, bevorzugt 0,1 bis 3,5 Massen-% und besonders bevorzugt 0,5 bis 2,5 Massen-% gelösten Chlorwasserstoff, bezogen auf das Gewicht des flüssigen Phosgen enthaltenden Stroms (iii-1), enthalten.

Der aus Schritt (iii) austretende flüssiges Phosgen enthaltende Strom (iii-1) hat in der Regel eine Temperatur von -40 bis 20 °C, bevorzugt -25 bis 15 °C und besonders bevorzugt -20 bis 10 °C. Dabei steht dieser Strom in der Regel bei Austritt aus dem Verfahrensschritt unter einem Druck von 1,00 bis 4,00 bar absolut, bevorzugt 1,01 bis 3,00 bar absolut und besonders bevorzugt von 1,02 bis 2,00 bar absolut. Als Austritt aus dem Verfahrensschritt für den flüssigen Phosgen enthaltenden Strom wird der Flüssigkeitsaustrittsstutzen aus dem oder den zu dieser Verfahrensstufe gehörigen Apparaten verstanden. Dabei wird der dort gemessene Druck um den hydrostatischen Druck der Flüssigkeitssäule in dem oder den Apparaten bereinigt.

Dieser Strom (iii-1) wird nun in **Schritt (iv)** partiell verdampft. Diese Teilverdampfung des Strom (iii-1) kann beispielsweise erfolgen durch:
- Einsatz eines geregelten Vorverdampfers (d. h. Wärmezufuhr etwa bei dem Druck der Destillationskolonne aus Schritt (v), dadurch Entstehung einer Dampfphase),
- geregelte Entspannungsverdampfung (teilweise Verdampfung durch Druckabsenkung) oder
- geregelte Überhitzung des flüssigen Zulaufstroms (iii-1) unter Druck (d.h . Erwärmen des flüssigen Zulaufs unter Druck auf eine Temperatur, die oberhalb der Siedetemperatur des Gemischs bei dem Druck der Destillationskolonne aus Schritt (v) liegt)

Die Vorwärmung und Teilverdampfung reduziert die Energiekosten, indem die Wärmezufuhr im Sumpf der Destillationskolonne aus Schritt (v) auf hohem Temperaturniveau teilweise durch Wärmezufuhr auf niedrigerem Temperaturniveau im Kolonnenzulauf ersetzt wird.

Die Vorwärmung und Teilverdampfung kann ggf. auch in mehreren Schritten (d. h. mit Hilfe mehrerer Apparate und/oder mehrerer Heizmedien) erfolgen.

Die Vorwärmung und Teilverdampfung kann insbesondere aufgrund des niedrigen bis mäßigen Temperaturniveaus ggf. auch durch Wärmeintegration mit geeigneten Prozessströmen und/oder Abwärmeströmen (Kondensat, niedriggespannter Dampf) erfolgen und ermöglicht damit einen besonders energieeffizienten Anlagenbetrieb, und zwar im Gegensatz zum Stand der Technik unabhängig von der Ausführung der Destillationskolonne aus Schritt (v) mit oder ohne Verstärkungsteil.

Erfolgt die Vorwärmung und Teilverdampfung in mehreren Schritten, müssen nicht alle Schritte bezüglich der Wärmeübertragung geregelt sein, was die Wärmeintegration durch geringe Rückwirkung auf andere Prozessschritte häufig erleichtert und dadurch zu einem robusteren Betriebsverhalten des Gesamtprozesses führt.

Auch wenn die Vorwärmung und Teilverdampfung in mehreren Schritten und ggf. mit Hilfe verschiedener Heizmedien erfolgt, können mehrere dieser Schritte in einem Apparat integriert sein. Reduzierte Apparatevolumina und weniger Verbindungen zwischen Apparaten und Rohrleitungen sind insbesondere bei phosgenführenden Apparaten sicherheitstechnisch vorteilhaft und führen zu geringeren Apparatekosten.

Die zuvor beschriebene erfindungsgemäße Vorgehensweise in Schritt (iv) ermöglicht es, den Druck in der Destillationskolonne des folgenden **Schritts (v)** (des Schrittes der Erzeugung gasförmigen Rückphosgens, das in Schritt (vi) in die Reaktion aus Schritt (i) rezykliert wird), durch Variation der Menge des dem in dieser Destillationskolonne zu trennenden Stoffgemisches direkt zugeführten Dampfes zu regeln. Unter dem *"Druck in der Destillationskolonne aus Schritt (v)"* wird im Rahmen dieser Erfindung der am Kopf der Kolonne gemessene Druck verstanden. Der Ausdruck *"dem in dieser Destillationskolonne zu trennenden Stoffgemisch direkt zugeführte Dampf"* bezieht sich dabei auf Phosgendampf-haltige Ströme, die mit dem in der Destillationskolonne zu trennenden Stoffgemisch *in stofflichen Kontakt treten,* bezieht sich also insbesondere auf den in Schritt (iv) erhaltenen Dampfanteil (es können aber noch, weiter unten erläutert wird, Phosgendampfanteile aus anderer Quelle vorhanden sein). Im Folgenden wird verkürzt auch der Ausdruck *"direkt zugeführter Dampf"* verwendet. Hiervon zu unterscheiden ist über eine indirekte Beheizung zugeführter Heizdampf, der nicht in *stofflichem* Kontakt mit dem zu trennenden Stoffgemisch in der Destillationskolonne steht.

Übliche Kolonnen-Druckregelungen verwenden im Falle dampfförmiger Kopfproduktentnahme (siehe Kister: "Distillation Operation" Kapitel 17.2) entweder (a) die Kopfproduktentnahmemenge selbst oder (b) die Kondensationsleistung als Stellgröße, seltener (c) gegen Atmosphäre oder Inertgas "atmende" Systeme oder (d) über den Sumpfverdampfer gestellte Regelkonzepte. Nachteile für den vorliegenden Anwendungsfall wären bei dieser Vorgehensweise des Standes der Technik: (a) Die Kopfproduktentnahme kann nicht gleichzeitig Regel- und Stellgröße sein; (b) die Verwendung der Kondensationsleistung als Stellgröße ist nicht schnell genug, insbesondere um schnell ansteigende Mengen Phosgen als Kopfproduktstrom zur Verfügung zu stellen; die Variante (c) ist wegen des Phosgenverlusts und der Notwendigkeit der Zuführung von Inertgas unvorteilhaft; (d) die Verwendung des Sumpfverdampfers als Stellgröße ist nicht schnell genug (siehe (b)).

Überraschend wurde jedoch gefunden, dass für den vorliegenden Anwendungsfall ein stabiler Kolonnendruck in Schritt (v) besonders gut dadurch geregelt werden kann, dass ein (mindestens teilweise) dampfförmiger, Phosgen-haltiger Zulaufstrom (das zweiphasige Verfahrensprodukt aus Schritt (iv)) zur Erzeugung des gasförmigen Rückphosgens als Stellgröße der Druckregelung verwendet wird.

Im vorliegenden Anwendungsfall sind die Parameter Kolonnendruck und Phosgenmengenentnahme am Kopf der Destillationskolonne von Schritt (v) eng aneinander gekoppelt, so dass die erfindungsgemäße Druckregelung, die beide Parameter parallel beeinflusst, besonders effektiv ist.

Im einfachsten Fall besteht der der Destillationskolonne aus Schritt (v) direkt zugeführte Dampf ausschließlich aus dem Dampfanteil des zweiphasigen Verfahrensprodukts aus Schritt (iv). In dieser Ausführungsform kann das zur Ergänzung des in Schritt (i) verbrauchten Phosgens zuzuführende - separat bereitgestelltes - Frischphosgen, falls dieses nicht bereits in Schritt (iii) zugeführt wurde und somit bereits Bestandteil des in dem zweiphasigen Verfahrensprodukt aus Schritt (iv) enthaltenden Phosgens ist, mit dem gasförmigen Phosgen-haltigen Strom aus Schritt (v) (d. h. dem "Rückphosgen"), ehe dieser der Reaktion von Schritt (i) zugeführt wird, vermischt werden. Es ist jedoch auch denkbar, das Rückphosgen und das Frischphosgen getrennt voneinander in die Reaktion von Schritt (i) zu führen. In beiden genannten Varianten kann Frischphosgen unverflüssigt, inklusive der ggf. enthaltenen Inertgase und des ggf. vorhandenen überschüssigen Kohlenstoffmonoxids aus seiner Herstellung direkt dem Reaktor zugeführt werden, falls die angestrebte Art der Weiterverwendung des im Prozess entstehenden Chlorwasserstoffs dies bezüglich seiner Reinheit zulässt. Anderenfalls kann das Frischphosgen - ebenfalls in beiden genannten Varianten - bevorzugt verflüssigt, in einem separaten Verdampfer verdampft und dann der Reaktion von Schritt (i) zugeführt werden.

Ebenfalls ist denkbar, Frischphosgen in flüssiger Phase, bspw. in einem Lösungsmittel gelöst, vorzugsweise dem in Schritt (ii) einzusetzenden Lösungsmittel gelöst, oder bevorzugt in reiner Form, in die Destillationskolonne von Schritt (v) einzuführen, z. B. in den Sumpf dieser Kolonne, wobei dieser dann die Funktion eines Phosgenverdampfers übernimmt. In einer bevorzugten Ausführungsform wird flüssiges Frischphosgen jedoch auf den Kopf dieser Kolonne aufgegeben, so dass das flüssige Frischphosgen die Funktion des Rücklaufs teilweise ersetzen kann. Sofern, obwohl grundsätzlich möglich, auf einen Kopfkondensator der Destillationskolonne aus Schritt (v) nicht verzichtet werden soll, kann Frischphosgen auch diesem Kopfkondensator verflüssigt als Rücklauf auf die Destillationskolonne zugeführt werden.

In einer bevorzugten Ausgestaltung des erfindungsgemäßen Verfahrens wird die Variation der Menge des der Destillationskolonne aus Schritt (v) zugeführten Dampfes durch Variation des Dampfanteils des in Schritt (iv) erzeugten zweiphasigen Verfahrensproduktes vorgenommen. Dies geschieht bevorzugt durch Variation der Temperatur des zweiphasigen Verfahrensprodukts aus Schritt (iv): Je höher dessen Temperatur ist, desto größer ist der Dampfanteil und umgekehrt. Die Temperatur des zweiphasigen Verfahrensprodukts kann bei Dampfbeheizung bspw. über eine geeignete Regelung des Heizdampfes eingestellt werden.

In einer anderen Ausführungsform des erfindungsgemäßen Verfahrens wird der Destillationskolonne aus Schritt (v) neben dem zweiphasigen Verfahrensprodukt aus Schritt (iv) dampfförmiges Frischphosgen zugeführt, d. h. der der Destillationskolonne direkt zugeführte Dampf umfasst den Dampfanteil des zweiphasigen Verfahrensprodukts aus Schritt (iv) und - separat bereitgestellten - Frischphosgendampf. Es ist nicht erforderlich, hierzu einen völlig dampfförmigen Phosgenstrom zu verwenden; vielmehr ist es auch möglich, der Destillationskolonne aus Schritt (v) einen weiteren zweiphasigen (d. h. flüssige und gasförmige Anteile enthaltenden) Strom zuzuführen. Auch in dieser Ausführungsform kann die Variation der Menge des der Destillationskolonne aus Schritt (v) zugeführten Dampfes durch Variation des Dampfanteils des in Schritt (iv) erzeugten zweiphasigen Verfahrensproduktes vorgenommen werden. Sie kann aber auch durch Variation des Dampfanteils des weiteren, Phosgendampf-haltigen Stroms vorgenommen werden (sofern der Strom nicht bereits völlig dampfförmig vorliegt, sondern stattdessen variierbare flüssige Anteil aufweist). Auch eine Kombination beider Maßnahmen ist denkbar.

Insbesondere bei dieser Ausführungsform (aber nicht auf diese beschränkt) kann es vorteilhaft sein, die Variation der Menge des der Destillationskolonne aus Schritt (v) direkt zugeführten Dampfes durch Variation der dieser Kolonne zugeführten Gesamtmenge (also der Summe aus dem flüssigen und dampfförmigen Anteil) an zweiphasigem Verfahrensprodukt aus Schritt (iv) und/oder durch Variation der dieser Kolonne zugeführten Gesamtmenge an separat bereitgestelltem, ggf. zweiphasigem, Phosgendampf-haltigen Strom vorzunehmen. Hierbei ist es bevorzugt, den Dampfanteil, ausgedrückt als Massenanteil des Gesamtmassenstroms aus Schritt (iv), des in Schritt (iv) erzeugten zweiphasigen Verfahrensproduktes im Wesentlichen konstant zu halten. *"Im Wesentlichen konstant"* bedeutet dabei, dass ein einmal eingestellter Dampfanteil maximal um einen Betrag schwankt, der 3 % des für den Dampfanteil angestrebten Soll-Werts entspricht. Wird für die Regelungsaufgabe auch oder ausschließlich die Variation der Gesamtmenge eines oben beschriebenen zusätzlich zugeführten, ggf. zweiphasigen, Phosgendampf-haltigen Stroms eingesetzt, so ist es bevorzugt, dessen Dampfanteil im Wesentlichen konstant zu halten. *"Im Wesentlichen konstant"* bedeutet dabei, dass ein einmal eingestellter Dampfanteil maximal um einen Betrag schwankt, der 3 % des für den Dampfanteil angestrebten Soll-Werts entspricht.

Diese Ausführungsform erlaubt es, auch das dampfförmige Frischphosgen als druckregelnden Zulaufstrom zu verwenden.

Durch die Druckregelung mittels des dampfförmigen Zulaufs der Destillationskolonne aus Schritt (v) kann auf den Kopfkondensator als Regeleinrichtung und auf den Verstärkungsteil verzichtet werden. Ein höherer Lösungsmittelanteil im Rückphosgen, als er im Stand der Technik in konkreten Ausführungsbeispielen offenbart ist, wird dabei bewusst in Kauf genommen und hat sich in der Praxis (bezüglich Reaktionsausbeute, Nebenprodukten etc.) als nicht nachteilig herausgestellt. Der Lösungsmittelanteil im Rückphosgen lässt sich mittels der Zulauftemperatur zur Destillationskolonne aus Schritt (v) gezielt einstellen. Durch Nachführung der Zulauftemperatur lässt sich der Lösungsmittelanteil im Rückphosgen auch bei geänderten Zulauf-Parametern (Zusammensetzung, Druck) konstant halten, was einen Vorteil gegenüber dem Stand der Technik (bspw. einer ungeregelten Strippkolonne) darstellt. Unter anderem führt eine höhere Zulauftemperatur zu einem erhöhten Lösungsmittelanteil im Rückführphosgen, reduziert aber andererseits auch die Energiekosten durch einen höheren AnteilWärmeintegration und Abwärmenutzung für die Zulauf-Beheizung und durch einen reduzierten Energiebedarf zum Aufheizen des Rückphosgenstroms. Eine Zulauftemperatur (d. h. die Temperatur des zweiphasigen Verfahrensprodukts aus Schritt (iv) bei Einspeisung in die Destillationskolonne von Schritt (v)) von 50 °C bis 150 °C, bevorzugt von 80 °C bis 120 °C, besonders bevorzugt von 90 °C bis 110 °C und ein Lösungsmittelanteil im gasförmigen Phosgen-haltigen Strom aus Schritt (v) von 1,0 Massen-% bis 15 Massen-%, bevorzugt von 2,5 Massen-% bis 10 Massen-%, besonders bevorzugt von 3,0 -Massen-% bis 7,5 Massen-%, haben sich bei einem Druck in der Destillationskolonne aus Schritt (v) von 10 mbar bis 1500 mbar über Umgebungsdruck (gemessen am Kopf der Kolonne) als besonders günstig herausgestellt.

Im erfindungsgemäßen **Schritt (vi)** wird der in Schritt (v) erhaltene gasförmige Phosgen-haltige Strom, ggf. nach Zugabe von Frischphosgen (siehe oben) in die Reaktion aus Schritt (i) zurückgeführt.

Die Aufarbeitung des in Schritt (ii) erhaltenen flüssigen, Lösungsmittel sowie Isocyanat enthaltenden Stroms (ii-2) zur Gewinnung des gewünschten Isocyanats in **Schritt (vi)** kann nach einem beliebigen Verfahren des Standes der Technik erfolgen. Bevorzugt erfolgt die Aufarbeitung destillativ. Dieser Schritt ist aus dem Stand der Technik hinreichend bekannt und wird bevorzugt wie in EP 1 371 635 B1, insbesondere in den Absätzen [0014] bis [0018], beschrieben durchgeführt.

Durch den Verzicht auf den Verstärkungsteil und bevorzugt auch den Kopfkondensator wird der Flüssig-Hold-up mit hohem Phosgenanteil in der Destillationskolonne aus Schritt (v) deutlich verringert. Bezogen auf die Gesamtanlage wird der Phosgen-hold-up dadurch signifikant, d. h. um ca. 10 % bis 20 % reduziert.

Durch den Verzicht auf den Kopfkondensator in den bevorzugten Ausführungsformen werden die Apparatekosten, die Anlagenkomplexität und gleichzeitig die Betriebskosten für Kondensationsenergie gesenkt.

Durch den Verzicht auf den Verstärkungsteil wird das Volumen der Destillationskolone aus Schritt (v) um bis zu 80 %, reduziert, was zu wesentlich geringeren Apparatekosten führt.

Weitere Vorteile des erfindungsgemäßen Verfahrens sind:
- Der Wegfall des Verstärkungsteils führt außerdem zu einem deutlich verringerten Druckverlust der Destillationskolone aus Schritt (v). Bei gleichem Kopfdruck sinken der Druck im Sumpf und die entsprechende Verdampfertemperatur, so dass der Verdampfer mit kleinerer Fläche dimensioniert werden kann.
- Der "Mittelsiederbauch" (z. B. Tetrachlormethan) in der Destillationskolone aus Schritt (v) wird deutlich reduziert, da Mittelsieder vermehrt über Kopf abgeführt werden und sich kaum anreichern. Ein Durchbrechen des Mittelsiederbauchs nach oben oder unten in der Kolonne führt zu Siedetemperaturschwankungen, da u.a. die Sumpftemperatur empfindlich auf Änderungen im Mittelsiederanteil reagiert. Geringere Mittelsiederanreicherung erleichtert daher die Regelbarkeit, insbesondere die Regelung des Sumpfprodukts auf Phosgenfreiheit, da die Siedetemperatur eines Stoffgemisches nur dann zur Berechnung und Regelung der Reinheit eines Strom (z. B. des Sumpfstroms) genutzt werden kann, wenn es sich um ein im Wesentlichen reines Zweistoffgemisch mit weit auseinander liegenden Siedepunkten handelt (z. B. Lösungsmittel und Phosgen). Ist sichergestellt, dass sich im Sumpf der Kolonne dauerhaft nur das Zweistoffgemisch Lösungsmittel/Phosgen befindet, wird die Kolonne in der Praxis zuverlässig genug betreibbar, um sie als Apparat zur Lösungsmittelentphosgenierung einzusetzen und auf dedizierte zusätzliche Apparate für diese Aufgabe zu verzichten.

- Das in Schritt (v) als flüssiges Sumpfprodukt abgezogene Gemisch, das im Wesentlichen aus Lösungsmittel besteht, wird bevorzugt in den Prozess zurückgeführt. Dieser Stoffstrom weist aufgrund seiner üblicherweise nahe des Siedepunktes liegenden Temperatur ein hohes Temperaturniveau auf, das für den Prozessschritt, in den es zurückgeführt wird (z. B. die Quenche) nicht notwendig oder sogar gar nicht erwünscht ist. Dieser Sumpfproduktstrom eignet sich daher in besonderer Weise für prozessinterne Wärmerückgewinnung unter Abkühlung dieses Sumpfproduktstroms, z. B. zur Verdampfung von flüssig vorliegendem Frischphosgen, zur Erwärmung des dampfförmigen zur Reaktion geführten Phosgenstroms, zur Verdampfung oder Überhitzung des Amins vor Eintritt in den Gasphasenreaktor, zur Überhitzung des abzuführenden Chlorwasserstoffstroms aus Schritt (iii), zur Vorwärmung oder Teilverdampfung in Schritt (iv), zur Beheizung von Sumpfverdampfern in der Aufarbeitungssequenz für das flüssige Quenche-Produkt oder an anderer Stelle in diesem Prozess.

Der erfindungsgemäße Vorteil der Möglichkeit der einfachen und effizienten Regelung des Drucks in der Destillationskolonne aus Schritt (v), insbesondere der Möglichkeit diesen Druck auch bei Änderungen der Randbedingungen auf einfache Weise im Wesentlichen konstant zu halten, kommt besonders zum Tragen, wenn die Produktion des gewünschten Isocyanats in mehreren (allgemein in *n,* wobei *n* eine natürliche Zahl ≥ 2 ist) parallel geschalteten Reaktionsstrecken jeweils umfassend die Schritte (i) und (ii) erfolgt, welchen eine geringere Anzahl (also maximal *n* - 1) Aufarbeitungsstrecken gemeinsam ist, in denen die Schritte (iii) bis (v) durchgeführt werden.

Besonders bevorzugt ist es, die in den *n* Reaktionsstrecken erhaltenen Verfahrensströme (ii-1)₁ bis (ii-1)*ₙ* zu vereinen und gemeinsam in einer einzigen Aufarbeitungsstrecke aufzuarbeiten. In dieser Ausführungsform ist es bevorzugt, den in der einzigen Aufarbeitungsstrecke erhaltenen gasförmigen Phosgen-haltigen Strom in Schritt (vi) in *n* Einzelströme aufzuteilen und auf die *n* Reaktionsstrecken zu verteilen.

Es ist jedoch auch möglich, die Reaktionsprodukte (ii-1) aus bspw. drei Reaktionsstrecken auf zwei Aufarbeitungsstrecken zu verteilen. Dies kann bspw. so geschehen, dass die einzelnen Ströme (ii-1)₁ bis (ii-1)₃ zunächst vereint und dann in zwei Ströme aufgeteilt werden. In dieser Ausführungsform ist es bevorzugt, die in den maximal (*n* - 1) Aufarbeitungsstrecken (im gewählten Beispiel in zwei Aufarbeitungsstrecken) jeweils erhaltenen gasförmigen Phosgenhaltigen Ströme auf *alle n* Reaktionsstrecken (im gewählten Beispiel also auf drei Reaktionsstrecken) zu verteilen. Dies kann so geschehen, dass die einzelnen Ströme zunächst vereint und dann auf die *n* Reaktionsstrecken verteilt werden.

## Patentansprüche

1. Kontinuierliches Verfahren zur Herstellung eines Isocyanats durch Phosgenierung des korrespondierenden primären Amins, umfassend die Schritte:
(i) Umsetzung des primären Amins mit einem Überschuss an Phosgen in der Gasphase;
(ii) Behandlung des in (i) erhaltenen Verfahrensprodukts mit einem Lösungsmittel bei einer Temperatur, die unterhalb der Siedetemperatur des Isocyanats und oberhalb der Zersetzungstemperatur des korrespondierenden Carbaminsäurechlorids liegt, unter Erhalt eines gasförmigen, Chlorwasserstoff sowie nicht umgesetztes Phosgen enthaltenden Stroms (ii-1) und eines flüssigen, Lösungsmittel sowie Isocyanat enthaltenden Stroms (ii-2);
(iii) Trennung des in Strom (ii-1) enthaltenen Chlorwasserstoffs und Phosgens unter Erhalt eines flüssigen Phosgen-haltigen Stroms (iii-1) und eines gasförmigen Chlorwasserstoff-haltigen Stroms (iii-2);
(iv) Partielle Verdampfung des flüssigen Phosgen-haltigen Stroms (iii-1) unter Erhalt eines zweiphasigen Verfahrensproduktes;
(v) Aufgabe des zweiphasigen Verfahrensproduktes aus Schritt (iv) am Kopf einer Destillationskolonne, welcher ein gasförmiger Phosgen-haltiger Strom über Kopf entnommen wird;
(vi) Rückführung des gasförmigen Phosgen-haltigen Stroms aus Schritt (v) in Schritt (i);
(vii) Aufarbeitung des in Schritt (ii) erhaltenen flüssigen, Lösungsmittel sowie Isocyanat enthaltenden Stroms (ii-2) zur Gewinnung des gewünschten Isocyanats.

2. Verfahren gemäß Anspruch 1, bei dem Schritt (iii) die Absorption von Phosgen in einem Lösungsmittel umfasst, das gemeinsam mit Phosgen im flüssigen Strom (iii-1) dem Schritt (iv) zugeführt wird.

3. Verfahren gemäß einem der vorstehenden Ansprüche, bei dem der Druck in der Destillationskolonne aus Schritt (v) durch Variation der Menge des dem in dieser Destillationskolonne zu trennenden Stoffgemisches direkt zugeführten Dampfes geregelt wird.

4. Verfahren gemäß Anspruch 3, bei dem der dem in der Destillationskolonne aus Schritt (v) zu trennenden Stoffgemisch direkt zugeführte Dampf neben
a) dem Dampfanteil des in Schritt (iv) erzeugten zweiphasigen Verfahrensproduktes zusätzlich
b) Phosgendampf aus einem weiteren, der Destillationskolonne zugeführten, ggf. zweiphasigen, Strom umfasst.

5. Verfahren gemäß Anspruch 4, bei dem die Variation der Menge des dem in der Destillationskolonne aus Schritt (v) zu trennenden Stoffgemisch zugeführten Dampfes
a) durch Variation der Gesamtmenge des dieser Kolonne zugeführten zweiphasigen Verfahrensproduktes aus Schritt (iv), oder
b) durch Variation der Gesamtmenge des dieser Kolonne zugeführten weiteren, ggf. zweiphasigen, Phosgendampf enthaltenden Stroms oder
c) durch beide Maßnahmen a) und b)
vorgenommen wird.

6. Verfahren gemäß Anspruch 5, bei dem der Dampfanteil
a) des in Schritt (iv) erzeugten zweiphasigen Verfahrensproduktes oder
b) des weiteren, ggf. zweiphasigen, Phosgendampf enthaltenden Stroms oder
c) beider Ströme a) und b)
im Wesentlichen konstant gehalten wird.

7. Verfahren gemäß Anspruch 4, bei dem die Variation der Menge des dem in der Destillationskolonne aus Schritt (v) zu trennenden Stoffgemisch zugeführten Dampfes
a) durch Variation des Dampfanteils des dieser Kolonne zugeführten zweiphasigen Verfahrensproduktes aus Schritt (iv), oder
b) durch Variation des Dampfanteils des dieser Kolonne zugeführten weiteren, in dieser Ausführungsform zweiphasigen, Phosgendampf enthaltenden Stroms oder
c) durch beide Maßnahmen a) und b)
vorgenommen wird.

8. Verfahren gemäß Anspruch 3, bei dem der dem in der Destillationskolonne aus Schritt (v) zu trennenden Stoffgemisch direkt zugeführte Dampf nur aus dem Dampfanteil des in Schritt (iv) erzeugten zweiphasigen Verfahrensproduktes besteht.

9. Verfahren gemäß Anspruch 8, bei dem die Variation der Menge des dem in der Destillationskolonne aus Schritt (v) zu trennenden Stoffgemisch zugeführten Dampfes durch Variation der Gesamtmenge des dieser Kolonne zugeführten zweiphasigen Verfahrensproduktes aus Schritt (iv) vorgenommen wird.

10. Verfahren gemäß Anspruch 9, bei dem der Dampfanteil des in Schritt (iv) erzeugten zweiphasigen Verfahrensproduktes im Wesentlichen konstant gehalten wird.

11. Verfahren gemäß Anspruch 8, bei dem die Variation der Menge des dem in der Destillationskolonne aus Schritt (v) zu trennenden Stoffgemisch direkt zugeführten Dampfes durch Variation des Dampfanteils des in Schritt (iv) erzeugten zweiphasigen Verfahrensproduktes vorgenommen wird.

12. Verfahren gemäß einem der vorstehenden Ansprüche, bei dem die Destillationskolonne in Schritt (v) nicht über einen Kopf-Kondensator verfügt.

13. Verfahren gemäß einem der vorstehenden Ansprüche, bei dem die Schritte (i) und (ii) in *n* Reaktionsstrecken parallel durchgeführt werden, wobei *n* eine natürliche Zahl ≥ 2 ist, und die in den *n* Reaktionsstrecken erhaltenen Verfahrensströme (ii-1)₁ bis (ii-1)*ₙ* in maximal (*n* - 1) Aufarbeitungsstrecken unter Durchführung der Schritte (iii) bis (v) aufgearbeitet werden.

14. Verfahren gemäß Anspruch 13, bei dem die in den *n* Reaktionsstrecken erhaltenen Verfahrensströme (ii-1)₁ bis (ii-1)*ₙ* vereint und gemeinsam in einer einzigen Aufarbeitungsstrecke aufgearbeitet werden.

15. Verfahren gemäß Anspruch 13, bei dem die in den maximal (*n - 1*) Aufarbeitungsstrecken jeweils erhaltenen gasförmigen Phosgen-haltigen Ströme auf alle *n* Reaktionsstrecken verteilt werden.

16. Verfahren gemäß Anspruch 14, bei dem der in der einzigen Aufarbeitungsstrecke erhaltene gasförmige Phosgen-haltige Strom in *n* Einzelströme aufgeteilt und auf die *n* Reaktionsstrecken verteilt wird.

## Claims

1. Continuous process for preparing an isocyanate by phosgenation of the corresponding primary amine, which comprises the steps:
(i) reaction of the primary amine with an excess of phosgene in the gas phase;
(ii) treatment of the process product obtained in (i) with a solvent at a temperature which is below the boiling point of the isocyanate and above the decomposition temperature of the corresponding carbamoyl chloride to give a gaseous stream (ii-1) containing hydrogen chloride and unreacted phosgene and a liquid stream (ii-2) containing solvent and isocyanate;
(iii)separation of the hydrogen chloride and phosgene present in the stream (ii-1) to give a liquid phosgene-containing stream (iii-1) and a gaseous stream (iii-2) containing hydrogen chloride;
(iv) partial vaporization of the liquid phosgene-containing stream (iii-1) to give a two-phase process product;
(v) introduction of the two-phase process product from step (iv) at the top of a distillation column, from which a gaseous phosgene-containing stream is taken off at the top;
(vi) recirculation of the gaseous phosgene-containing stream from step (v) to step (i);
(vii)work-up of the liquid stream (ii-2) containing solvent and isocyanate obtained in step (ii) to isolate the desired isocyanate.

2. Process according to Claim 1, wherein step (iii) comprises the absorption of phosgene in a solvent which is fed together with phosgene in the liquid stream (iii-1) into step (iv).

3. Process according to either of the preceding claims, wherein the pressure in the distillation column of step (v) is regulated by variation of the amount of the vapour introduced directly into the mixture to be separated in this distillation column.

4. Process according to Claim 3, wherein the vapour introduced directly into the mixture to be separated in the distillation column of step (v) comprises
a) the vapour fraction of the two-phase process product produced in step (iv) together with
b) phosgene vapour from a further, optionally two-phase, stream introduced into the distillation column.

5. Process according to Claim 4, wherein the variation of the amount of the vapour introduced into the mixture to be separated in the distillation column of step (v) is carried out
a) by variation of the total amount of the two-phase process product from step (iv) which is introduced into this column, or
b) by variation of the total amount of the further, optionally two-phase, stream containing phosgene vapour which is introduced into this column or
c) by both measures a) and b).

6. Process according to Claim 5, wherein the proportion of vapour
a) in the two-phase process product produced in step (iv) or
b) in the further, optionally two-phase, stream containing phosgene vapour or
c) in both streams a) and b)
is kept essentially constant.

7. Process according to Claim 4, wherein the variation of the amount of the vapour introduced into the mixture to be separated in the distillation column of step (v) is carried out
a) by variation of the proportion of vapour in the two-phase process product from step (iv) which is introduced into this column, or
b) by variation of the proportion of vapour in the further, in this embodiment two-phase, stream containing phosgene vapour which is introduced in this column or
c) by both measures a) and b).

8. Process according to Claim 3, wherein the vapour introduced directly into the mixture to be separated in the distillation column of step (v) consists entirely of the vapour fraction of the two-phase process product produced in step (iv).

9. Process according to Claim 8, wherein the variation of the amount of the vapour introduced into the mixture to be separated in the distillation column of step (v) is carried out by variation of the total amount of the two-phase process product from step (iv) which is introduced into this column.

10. Process according to Claim 9, wherein the proportion of vapour in the two-phase process product produced in step (iv) is kept essentially constant.

11. Process according to Claim 8, wherein the variation of the amount of the vapour introduced directly into the mixture to be separated in the distillation column of step (v) is carried out by variation of the proportion of vapour in the two-phase process product produced in step (iv).

12. Process according to any of the preceding claims, wherein the distillation column in step (v) does not have an overhead condenser.

13. Process according to any of the preceding claims, wherein the steps (i) and (ii) are carried out in parallel in *n* reaction lines, where *n* is a natural number ≥ 2, and the process streams (ii-1)₁ to (ii-1)*ₙ* obtained in the *n* reaction lines are worked up in not more than (*n*-1) work-up lines in which the steps (iii) to (v) are carried out.

14. Process according to Claim 13, wherein the process streams (ii-1)₁ to (ii-1)*ₙ* obtained in the *n* reaction lines are combined and worked up together in a single work-up line.

15. Process according to Claim 13, wherein the gaseous phosgene-containing streams obtained in each case in the not more than (*n*-1) work-up lines are distributed over all *n* reaction lines.

16. Process according to Claim 14, wherein the gaseous phosgene-containing stream obtained in the single work-up line is divided into *n* individual streams and distributed over the *n* reaction lines.

## Revendications

1. Procédé continu pour la préparation d'un isocyanate par phosgénation de l'amine primaire correspondante, comprenant les étapes :
(i) transformation de l'amine primaire avec un excès de phosgène en phase gazeuse ;
(ii) traitement du produit de procédé obtenu dans (i) par un solvant à une température qui se situe sous la température d'ébullition de l'isocyanate et au-dessus de la température de décomposition du chlorure d'acide carbamique correspondant, avec obtention d'un flux gazeux (ii-1) contenant du chlorure d'hydrogène ainsi que du phosgène non transformé et d'un flux liquide (ii-2), contenant du solvant ainsi que de l'isocyanate ;
(iii) séparation du chlorure d'hydrogène et du phosgène contenu dans le flux (ii-1) avec obtention d'un flux liquide (iii-1) contenant du phosgène et d'un flux gazeux (iii-2) contenant du chlorure d'hydrogène ;
(iv) évaporation partielle du flux liquide (iii-1) contenant du phosgène avec obtention d'un produit de procédé à deux phases ;
(v) introduction du produit de procédé à deux phases de l'étape (iv) en tête d'une colonne de distillation dont on prélève un flux gazeux contenant du phosgène via la tête ;
(vi) recyclage du flux gazeux contenant du phosgène de l'étape (v) dans l'étape (i) ;
(vii) traitement du flux liquide (ii-2), contenant du solvant ainsi que de l'isocyanate, obtenu dans l'étape (ii) pour la production de l'isocyanate souhaité.

2. Procédé selon la revendication 1, dans lequel l'étape (iii) comprend l'absorption de phosgène dans un solvant qui est introduit ensemble avec le phosgène dans le flux liquide (iii-1) de l'étape (iv).

3. Procédé selon l'une quelconque des revendications précédentes, dans lequel la pression dans la colonne de distillation de l'étape (v) est régulée par variation de la quantité de la vapeur introduite directement dans cette colonne de distillation dans le mélange de substances à séparer.

4. Procédé selon la revendication 3, dans lequel la vapeur introduite directement dans la colonne de distillation de l'étape (v) dans le mélange de substances à séparer comprend
a) la proportion de vapeur du produit de procédé à deux phases généré dans l'étape (iv) et
b) la vapeur de phosgène d'un autre flux introduit dans la colonne de distillation, le cas échéant à deux phases.

5. Procédé selon la revendication 4, dans lequel la variation de la quantité de la vapeur introduite dans la colonne de distillation de l'étape (v) dans le mélange de substances à séparer est réalisée
a) par variation de la quantité totale du produit de procédé de l'étape (iv) à deux phases introduit dans cette colonne ou
b) par variation de la quantité totale de l'autre flux, le cas échéant à deux phases, contenant de la vapeur de phosgène, introduit dans cette colonne ou
c) par les deux mesures a) et b).

6. Procédé selon la revendication 5, dans lequel la proportion de vapeur
a) du produit de procédé à deux phases généré dans l'étape (iv) ou
b) de l'autre flux, le cas échéant à deux phases, contenant de la vapeur de phosgène, ou
c) des deux flux a) et b)
est maintenue essentiellement constante.

7. Procédé selon la revendication 4, dans lequel la variation de la quantité de la vapeur introduite dans la colonne de distillation de l'étape (v) dans le mélange de substances à séparer est réalisée
a) par variation de la proportion de vapeur du produit de procédé de l'étape (iv) à deux phases introduit dans cette colonne ou
b) par variation de la proportion de vapeur de l'autre flux, le cas échéant à deux phases, contenant de la vapeur de phosgène dans ce mode de réalisation, introduit dans cette colonne ou
c) par les deux mesures a) et b).

8. Procédé selon la revendication 3, dans lequel la vapeur introduite directement dans la colonne de distillation de l'étape (v) dans le mélange de substances à séparer est constituée uniquement par la proportion de vapeur du produit de procédé à deux phases généré dans l'étape (iv).

9. Procédé selon la revendication 8, dans lequel la variation de la quantité de la vapeur introduite dans la colonne de distillation de l'étape (v) dans le mélange de substances à séparer est réalisée par variation de la quantité totale du produit de procédé à deux phases de l'étape (iv) introduit dans cette colonne.

10. Procédé selon la revendication 9, dans lequel la proportion de vapeur du produit de procédé à deux phases généré dans l'étape (iv) est maintenue de manière essentiellement constante.

11. Procédé selon la revendication 8, dans lequel la variation de la quantité de la vapeur introduite directement dans la colonne de distillation de l'étape (v) dans le mélange de substances à séparer est réalisée par variation de la proportion de vapeur du produit de procédé à deux phases généré dans l'étape (iv) .

12. Procédé selon l'une quelconque des revendications précédentes, dans lequel la colonne de distillation dans l'étape (v) ne dispose pas d'un condenseur de tête.

13. Procédé selon l'une quelconque des revendications précédentes, dans lequel les étapes (i) et (ii) sont réalisées en parallèle dans *n* zones de réaction, *n* étant un nombre naturel ≥ 2 et les flux de procédé (ii-1)₁ à (ii-1)*ₙ* obtenus dans les *n* zones de réaction sont traités dans au maximum (n - 1) zones de traitement avec réalisation des étapes (iii) à (v).

14. Procédé selon la revendication 13, dans lequel les flux de procédé (ii-1)₁ à (ii-1)*ₙ* obtenus dans les *n* zones de réaction sont rassemblés et traités conjointement dans une seule zone de traitement.

15. Procédé selon la revendication 13, dans lequel les flux gazeux contenant du phosgène obtenus à chaque fois dans les au maximum (*n* - 1) zones de traitement sont répartis sur toutes les *n* zones de réaction.

16. Procédé selon la revendication 14, dans lequel le flux gazeux contenant du phosgène obtenu dans l'unique zone de traitement est réparti en *n* flux individuels et réparti sur les *n* zones de réaction.
